Europäisches Patentamt

European Patent Office

Office européen des brevets

(11) Publication number: 0 384 997 A1

## EUROPEAN PATENT APPLICATION

(21) Application number: 89300850.8

(51) Int. Cl.5: C01B 33/34, B01J 29/18

(22) Date of filing: 27.01.89

(43) Date of publication of application:
05.09.90 Bulletin 90/36

(84) Designated Contracting States:
BE DE FR GB IT NL

(71) Applicant: Council of Scientific and Industrial Research
Rafi Marg
New Delhi 110 001(IN)

(72) Inventor: Chandwadkar, Asha Jeevan
National Chemical Laboratory
Pune Maharashtra(IN)
Inventor: Ratnasamy, Paul
National Chemical Laboratory
Pune Maharashtra(IN)

(74) Representative: Clifford, Frederick Alan et al
MARKS & CLERK 57/60 Lincoln's Inn Fields
London WC2A 3LS(GB)

(54) Process for the preparation of high silica large-port mordenites.

(57) The process disclosed is for the preparation of a high-silica, large port mordenite which is particularly useful as sorbent or as a catalyst e.g. for hydrocarbon conversions such as paraffin cracking, isomerisation, alkylation, hydrocracking, aromatisation etc. The high-silica, large port mordenite is represented in terms of mole ratios of oxides by the formula

$R_2O : M_2O : Al_2O_3 : (15-60) SiO_2 : Z H_2O$

wherein R is tetraethyl ammonium, M is an alkali metal and Z is above 7. In the process, a reaction mixture of tetraethyl ammonium bromide and sources of alkali, alumina and silica with water is formed and thereafter the reaction mixture is maintained at a temperature between $100°C$ and $200°C$ for a sufficient period of time.

EP 0 384 997 A1

## PROCESS FOR THE PREPARATION OF HIGH SILICA LARGE PORT MORDENITES

This invention relates to a process for the preparation of zeolites. More particularly, it relates to the .aration of large-port, high silica mordenites. More specifically, the present invention relates to a process for the synthesis of high-silica, large port mordenites using tetra ethyl ammonium compounds as templating agents.

BACKGROUND OF THE INVENTION

Zeolites are crystalline alumino-silicates with a three dimensional network of alumina and silica tetrahedra interlinked in the crystalline structure through oxygen atoms. Mordenite is a form of zeolite that occurs naturally and has also been synthesised. It has the general formula $Na_2O : Al_2O_3 : 9\text{-}10\ SiO_2 : 7H_2O$. The aluminosilicate framework involves a chain containing 5-membered rings of tetrahedra. They are inter-spaced with 4-membered rings. The chains consist of 5 rings of $SiO_4$ tetrahedra and single $AlO_4$ tetrahedra. The 5 and 4 membered rings form twisted 12 rings which form a system of channels with the free diameter of 6.6A°. The walls of these channels are formed by 5,6 and 8 membered rings forming inter-connecting channels with diameter of 2.8A°. Even though the diameter of the channels of mordenite are capable of adsorbing and catalysing reactions of molecules like benzene, xylene, etc., natural and synthetic mordenite often fail in the capacity of adsorbing those cyclic compounds because of stacking faults in the crystal structure. Such mordenites are known as small port mordenites. Mordenites which are free from stacking faults in the crystal structure and are able to adsorb significant amounts of cyclic molecules like benzene, usually exceeding about 9 % by weight of the mordenite, are known as large port mordenites. Mordenite zeolites having a silica to alumina ratio greater than 10 in the zeolite lattice are known as high silica mordenites. For many catalytic applications mordenites with a high silica to alumina ratio in the lattice as well as large port characteristics are preferred.

In prior art, processes for the preparation of high silica mordenites are known. U.S. Patent No.4,585,640 discloses a method for making high silica mordenite using synthesis directing organic dyes. U.S.Patent No.4,377,502 disclosed a process for the synthesis of mordenite using oxygen containing monomeric organic templates like ethanolamine, diethanolamine, dioxane and crown ethers. European Patent No.14023 discloses a process for the synthesis of high silica mordenite using neopentylamines. European Patent No.80615 discloses a method for the preparation of high silica mordenite using a methyl-piperidinium compound as the organic base.

While all the above-mentioned processes provide methods for the preparation of high silica mordenites, they do not always lead to mordenite zeolites having large port characteristics capable of adsorbing cyclic organic compounds like benzene in excess of 9 % by weight and thereby of utility in catalytic processes involving the said cyclic organic compounds. The specifically disclosed high silica mordenite of Example 9 of U.S.Patent No.4,585,640 for example, adsorbed only 4.1% of benzene at a benzene partial pressure of 70 torr and ambient temperature and is, hence, of limited utility in catalytic hydrocarbon conversions involving cyclic organic compounds.

High silica, large port mordenites can be prepared from a small port, low silica mordenites with a silica to alumina ratio of about 10 by removal of alumina from the crystal lattice by indirect, post synthesis, chemical treatment methods. U.S. patent No.4,022,681 discloses a method of treating sodium mordenite with an acid to provide a hydrogen-mordenite with a silica to alumina molar ratio between 15 and 70. French Patent No. FR 2,477,903 discloses a method of preparing mordenite catalyst with silica to alumina ratio above 14 and pores of 7 to 7.5A° from sodium mordenite with a silica to alumina ratio of 10 and pores of about 5A° by heat treatment with a strong acid and then treating with alkali or alkaline earth cations. U.S.Patent Nos.4,182,692 and 4,232,181 disclose a process for acid leaching and calcining an ammonium exchanged mordenite to obtain a mordenite with superior adsorption properties.

The hereinbefore mentioned indirect post-synthesis dealuimination methods for the preparation of light silica, large port mordenites have certain limitations mentioned hereinbelow. The use of concentrated acid solutions, in the process of removal of alumina from the mordenite structure, necessitates, due to the corrosive nature of said acidic solutions, the use of special expensive alloy metals for the vessels wherein the said acidic solutions are contacted with mordenite zeolite. In addition, the safe disposal of such acidic solutions after the preparation of the said large port mordenite, causes severe problems in complying with environmental safety regulations.

In view of the hereinbefore mentioned factors, it would be advantageous to provide a process for the preparation of high silica large port mordenite that is both direct and efficient and does not involve the removal of alumina from preformed mordenite by means of acidic solutions or other chemical treatment.

## SUMMARY OF THE INVENTION

The present invention relates to a process for the preparation of high silica, large port mordenite by direct synthesis methods. Broadly stated, the present invention contemplates the provision of a method for making high silica, large port mordenite comprising the steps of :

(a) providing a mixture comprising water, a source of silica, source of alumina, a source of alkali and tetraethylammonium bromide, the silica to alumina molar ratio in the said mixture being at least above 30, and

(b) maintaining said mixture at a temperature of 100 to about 200 $^\circ$ C for a sufficient time to produce the hereinbefore mentioned mordenite.

Table 1 shows X-ray data of the high silica, large port mordenite of this invention in the as-synthesised form. X-ray data were determined by standard technique using $CuK_2$ radiation. The framework IR spectra of the mordenite is shown in Table 2.

## BRIEF DESCRIPTION OF THE INVENTION

The present invention is a method of producing a crystalline, high silica, large port mordenite using tetraethyl ammonium bromide as an organic template.

The mordenite of this invention has the following composition in terms of molar oxides

$R_2O : M_2O : Al_2O_3 : (15-60) SiO_2 : ZH_2O$

Wherre M is an alkali metal ion and R is tetraethylammonium bromide and Z is from 7 to 10. It is characterised by an X-ray diffraction pattern as set forth in Table 1 and a framework infrared spectra as set forth in Table 2.

The mordenite of this invention is prepared by crystallizing an aqueous mixture, at a controlled pH, of a source of alkali metal, an oxide of aluminium, an oxide of silicon and tetraethyl ammonium bromide Typically, the mole ratios of the various reactants can be varied to produce the mordenite of this invention. Specifically, the mole ratios of the initial reactant concentrations are indicated below:

$SiO_2/Al_2O_3 = 10-60$

$M_2O/SiO_2 = 30-100$

$H_2O/SiO_2 = 30-100$

$R_2O/SiO_2 = 0.01-1.0$

where R is tetraethyl ammonium bromide and M is an alkali metal.

By regulation of the quantity of aluminium in the reaction mixture, it is possible to vary the $SiO_2/Al_2O_3$ molar ratio in the final product in a range from about 15 to 60.

The source of silica in the reaction mixture used in the preparation of the mordenite of this invention can be silicon dioxide, silica sol, sodium silicate or silicon alkoxides. The silica sols are aqueous colloidal dispersions containing colloidal silica particles. A preferred source of silicon is silicon dioxide.

The source of aluminium in the reaction mixture used in the preparation of the mordenite of the present invention can be sodium aluminate, alkoxides, hydroxides and oxides of aluminium as well as hydrates of aluminium. A preferred source of aluminium is sodium aluminate.

The source of alkali metal can include the oxides, hydroxides, inorganic and organic salts of alkali metals. A preferred source of alkali metal is sodium hydroxide.

The mordenite of the present invention can be prepared by mixing in any order the sources of silica, aluminium, alkali metal, tetraethyl ammonium bromide and water. The resulting mixture is then heated in an autoclave and maintained at a temperature from 100 to 200 $^\circ$ C for a period of time sufficient to crystallise the desired mordenite material. After crystallisation is over, the contents of the autoclave are quenched with cold water and are filtered, washed thoroughly with deionized water and dried at 120 $^\circ$ C for 12 hours. Then, this material, so obtained, is calcined at 480 $^\circ$ C for 16 hours. Then, in order to remove the organics from the crystalline solid material to yield the sodium form of the high silica, large port mordenite of the present invention. For many applications in the chemical industry, especially as a catalyst, the sodium form of the mordenite, obtained as hereinabove described, is converted into the catalytically active protonic form by ion exchange with an aqueous solution contaiing ammonium ions by conventional ion exchange procedures, filtering the resultant ammonium form of the mordenite and drying and calcining the same at a temperature

3

above 400°C. Even though the protonic form of the high silica large port mordenite thus obtained may be directly used as a catalyst, it is desirable, in many large scale applications, to enhance its mechanical strength and ease of handling by admixing it with a suitable binder material and converting into a suitable shape such as cylindrical extrudates, spheres, etc. Examples of suitable binder material which impart improved mechanical properties to the mordenite of this invention include silica, alumina, silica-alumina, clay minerals such as bentonite, kaolinite and mixtures thereof.

An advantageous feature of the process of the present invention is that the organic template used therein, namely, tetraethylammonium bromide is removed from the mordenite structure at a relatively low temperature of 400°C of heat treatment in air thereby facilitating the preparation of the catalytically active protonic form of the mordenite at less severe conditions of heat treatment.

In a preferred embodiment of the present invention, the mordenite material has the chemical composition in terms of mole ratios by oxides as follows:

$R_2O : Na_2O : Al_2O_3 : (15-60) SiO_2 : ZH_2O$

where R is tetraethyl ammonium and Z varies between 8 to 10.

In another embodiment of the present invention, the mordenite material has X-ray diffraction pattern substantially as set forth in Table 1 and an infrared spectra as shown in Table 2.

In another preferred embodiment of the process of the present invention, the mordenite has a silica to alumina molar ratio above 15.

In yet another preferred embodiment of the present invention, the mordenite adsorbs at least 9 % by weight of benzene when the adsorption of benzene is carried out from the vapour phase at a relative vapor pressure of benzene of 0.5 at a temperature of 25°C.

In one embodiment of the process of the present invention the reaction mixture for the preparation of the mordenite is usually reacted under autogeneous pressure of water in a closed vessel at temperature between 100 and 200°C until crystals of mordenite form which can be from 10 to 100 hrs depending on the reactant composition and the operating temperature. Agitation is optional but is preferred since it reduces the reaction time. At the end of the reaction, the solid phase is collected on a filter and washed with water and is then ready for further steps such as drying dehydration and ion exchange. If the product of the reaction contains alkali metal ions, these have to be at least partly removed in order to prepare the catalytically active protonic form of the high silica large port mordenite and this can be done by ion exchange with a solution of ammonium compound such as ammonium chloride or nitrate. The ion exchange can also be done with a solution of a strong acid. The ion exchange can be carried out by slurrying once or several times with ion exchange solution. The mordenite is usually calcined after ion exchange or before ion exchange but this may also be carried out during ion exchange if the latter is done in a number of stages.

The high silica large port modenite of the present invention may be used as sorbent or as a catalyst e.g. for hydrocarbon conversions such as paraffin cracking, isomerisation, alkylation, hydrocracking and aromatisation, etc.

Table - 1

| X-ray diffraction pattern of high silica,large port mordenite | |
|---|---|
| d | Relative intensity |
| 13.60 | m |
| 10.21 | m |
| 9.05 | s |
| 6.52 | s |
| 6.37 | m |
| 6.02 | w |
| 5.76 | w |
| 4.95 | w |
| 3.99 | s |
| 3.80 | w |
| 3.61 | w |
| 3.42 | vs |
| 3.35 | s |
| 3.20 | ms |
| 2.85 | m |
| 2.55 | w |
| 2.42 | w |
| vs - very strong<br>s - strong<br>ms - medium strong<br>m - medium<br>w - weak | |

Table - 2

| Framework infrared vibrations frequencies of high silica, large port mordenite | |
| --- | --- |
| Frequency, cm$^{-1}$ | Intensity |
| 1230 | w |
| 1060 | s |
| 798 | w |
| 690 | w |
| 625 | w |
| 582 | w |
| 455 | ms |
| 375 | vw |
| s - strong w - weak vw - very weak ms - medium strong | |

The process of this invention is further described with the following examples which are for illustrative purposes only and not to be construed as limitations.

Example 1

An aqueous reaction mixture was first prepared consisting of the following parts : 16685 grams of water, 1865 grams of microcrystalline silica, 630 grams of tetraethyl ammonium bromide and 482 grams of sodium hydroxide. A second reaction mixture was prepared using 2000 grams of water and 233 grams of sodium aluminate. The two mixtures were mixed together in a stainless steel autoclave with the result being the formation of a white gel. The molar ratio of the oxides in the gel was the following

$R_2O/SiO_2 = 0.05$
$SiO_2/Al_2O_3 = 30$
$H_2O/SiO_2 = 35$
$OH/SiO_2 = 0.5$

Wherein R is tetraethyl ammonium. The gel was first homogenised by vigorous stirring. The autoclave was then heated to a temperature of 180°C and maintained at this temperature for 24 hrs. At the end of this time the autoclave was opened and its contents quenched in cold water. The crystalline solids were separated from the mother liquor by filtration, washed thoroughly with water and then air dried at room temperature. 1487 grams of crystalline solid were thus recovered. The X-ray diffraction pattern and IR spectra of the solid were similar to those shown in Tables 1 and 2, respectively, establishing the solid to be mordenite. Its chemical composition was found in terms of mole ratios of oxides to be

$R_2O : Na_2O : Al_2O_3 : 20.5 SiO_2 : 8.1 H_2O$

wherein R is tetraethyl ammonium, establishing the mordenite to be a high silica mordenite.

Example 2

The crystalline solids obtained in Example 1 were heat treated for 10 hrs at 120°C in an atmosphere of flowing dry air. They were then further heat treated in the same atmosphere for a further period of 10 hours but at a temperature of 480°C. The crystalline solid obtained at this stage had a X-ray diffraction pattern and infrared spectra substantially similar to those shown in Tables 1 and 2 and characteristic of mordenite. The adsorption characteristics of this mordenite were evaluated using a conventional Mcbain gravimetric adsorption system. The solid was found to adsorb benzene to the extent of 9.8 % of its dry weight, thereby establishing it to possess large pores of diameter greater than 7A° when synthesised as described in Example 1, and hence to be a high silica, large port mordenite.

## Example 3

The process of Example 1 was repeated except that the molar ratio of the oxides in the reaction gel was

$R_2O/SiO_2$ = 0.12
$SiO_2/Al_2O_3$ = 60
$H_2O/SiO_2$ = 42
$OH/SiO_2$ = 0.64

where R is tetraethyl ammonium. The molar composition of the crystalline solid dried at room temperature was found to be

$R_2O : Na_2O : Al_2O_3 ; 53.4 SiO_2 : 8.5 H_2O$

wherein R is tetraethyl ammonium. After heat treatment above 400°C, as described in Example 1, the material was found to adsorb 10.9 % by weight of benzene.

## Example 4

The present example describes the process of conversion of the high silica, large port mordenite obtained in Example 1 after heat treatment above 400°C, into the catalytically active, protonic form 100 gram of the solid material obtained after heat treatment above 400°C and consisting of the sodium form of mordenite was contacted successively with 500 CC of an aqueous one molar solution of ammonium nitrate and filtered till the sodium content in the filtrate was less than 100 ppm. The crystalline solid material was then filtered, dried at 120°C for 10 hrs and then calcined at 480°C for 10 hrs. 10 grams of this material was mixed with 10 grams of alumina hydrate and extruded into cylindrical pellets. These pellets were tested for their catalytic activity in the disproportionation of toluene at the following reaction conditions:

Temp °C = 490°C
$H_2$/toluene = 4, molar
LHSV = 2
Pressure = 40 bar
Reactor = Downflow integral type

under the above conditions, 45 % by weight of toluene was converted into a mixture of benzene and xylenes in a molar ratio of 1.05.

## Claims

1. A process for the preparation of high silica, large port mordenite characterized by forming a reaction mixture with water, tetraethyl ammonium bromide, a source of alkali metal, a source of alumina and a source of silica and maintaining the said reaction mixture at a temperature of between 100 and 200°C for a sufficient period of time to produce a high silica large port mordenite represented in terms of mole ratios of oxides by the formula

$R_2O : M_2O : Al_2O_3 : (15-60) SiO_2 : Z H_2O$

wherein R is tetraethyl ammonium, M is an alkali metal and Z is above 7.

2. A process as claimed in Claim 1, characterized in that the reaction is effected so that molar ratio of silica to alumina in the said mordenite is from 20 to 40.

3. A process as claimed in Claims 1 or 2 in which the said alkali metal is sodium.

4. A process as claimed in Claims 1, 2 or 3 characterized in that Z is from 8 to 10.

5. A process as claimed in any one of Claims 1 to 4 characterized in that the source of alkali is sodium hydroxide.

6. A process as claimed in any one of Claims 1 to 5 characterized in that the source of silicon is silicon dioxide.

7. A process as claimed in any one preceding claims characterized in that the source of alumina is sodium aluminate.

EP 0 384 997 A1

8. A process as claimed in any one preceding claim characized in that the reaction mixture used in the preparation of the said mordenite has a molar composition, in terms of ratios of oxides, as under:

$SiO_2/Al_2O_3$ = 30 to 80

$H_2O/SiO_2$ = 30 to 100

$OH/SiO_2$ = 0.1 to 1.0

$R_2O/SiO_2$ = 0.01 to 1.0

wherein R is tetraethyl ammonium bromide.

9. A process as claimed in any one preceding claim characterized in that the reaction mixture is maintained at a temperature from 100 to 200°C for a period from 10 to 100 hours:

10. A process as claimed in any one preceding claim characterized in that the solid mordenite product obtained from the reaction mixture is separated from the mother liquor; washed; dried; heat treated in an inert or oxidising atmosphere at a temperature above 400°C from 10 to 30 hours; ion exchanged with an aqueous solution of an ammonium salt; and again heat treated in inert or oxidising atmosphere at a temperature above 400°C from 10 to 30 hours, to provide the catalytically active protonic form of the said mordenite.

11. A process as claimed in Claim 10 characterized in that it is effected to yield mordenite adsorbs at least 9% by weight of benzene at 25°C and at a relative partial pressure of benzene of 0.5.

12. A process as claimed in any one preceding claim characterized in that the mordenite has X-ray diffraction pattern substantially as set forth in Table 1 and an infrared spectra as shown in Table 2.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 107, no. 10, 7th September 1987, page 157, abstract no. 80499k, Columbus, Ohio, US; & JP-A-62 113 715 (NEW FUEL OIL DEVELOPMENT TECHNOLOGY RESEARCH ASSOCIATION) 25-05-1987 * Abstract * --- | 1,3,5-7 ,9 | C 01 B 33/34 B 01 J 29/18 |
| A | IDEM --- | 8 | |
| A | US-A-4 205 052 (L.D. ROLLMANN et al.) * Examples 1,11 * --- | 1,3,9 | |
| A | US-A-4 703 025 (G.T. KOKOTAILO et al.) * Example 3 * --- | 1,3,5-7 ,9 | |
| D,A | EP-A-0 080 615 (MONTEDISON S.p.A.) * Claims 7,8 * ----- | 10 | |

TECHNICAL FIELDS SEARCHED (Int. Cl.5)

C 01 B 33/00

The present search report has been drawn up for all claims

| Place of search | Date of completion of the search | Examiner |
|---|---|---|
| THE HAGUE | 10-03-1989 | BREBION J.CH. |